# EUROPEAN PATENT SPECIFICATION

(11) **EP 2 520 255 B1**
(45) Date of publication and mention of the grant of the patent: **15.06.2016**
(21) Application number: 12170854.9
(22) Date of filing: 21.11.2006
(51) Int. Cl.: A61F 2/46

(54) **Methods for treating facet joints, uncovertebral joints, costovertebral joints and other joints**
Verfahren zur Behandlung von Facettengelenken, Uncovertebralgelenken, Costovertebralgelenken und anderen Gelenken
Procédés de traitement de facettes articulaires lombaires, d'articulations uncovertébrales, d'articulations costovertébrales et d'autres articulations

(30) Priority: 21.11.2005 US 740323 P
(43) Date of publication of application: 07.11.2012
(62) Divisional of application: 06838227.4
(73) Proprietor: Vertegen, Inc., Lexington, MA 02421 (US)
(72) Inventor: Lang, Philipp, Lexington, MA 02421 (US)
(74) Representative: Grund, Martin

(56) References cited:
- WO-A1-2006/065774
- WO-A2-00/19911

## Description

### CROSS-REFERENCE TO RELATED APPLICATIONS

The current application claims the benefit of U.S. provisional patent application 60/740323 filed on November 21, 2005, entitled "Devices and Methods for Treating Facet Joints, Uncovertebral Joints, Costovertebral Joints and Other Joints". This application is a continuation-in-part of U.S. Ser. No. 10/997,407, filed November 24, 2004 entitled "PATIENT SELECTABLE KNEE JOINT ARTHROPLASTY DEVICES," which is a continuation-in-part of U.S. Ser. No. 10/752,438, filed Jan. 5, 2004 which is a continuation-in-part of U.S. application Ser. No. 10/724,010 filed Nov. 25, 2003 entitled "P ATIENT SELECTABLE JOINT ARTHROPLASTY DEVICES AND SURGICAL TOOLS FACILITATING INCREASED ACCURACY, SPEED AND SIMPLICITY IN PERFORMING TOTAL AND PARTIAL JOINT ARTHROPLASTY," which is a continuation-in-part of U.S. Ser. No. 10/305,652 entitled "METHODS AND COMPOSITIONS FOR ARTICULAR REPAIR ," filed Nov. 27, 2002, which is a continuation-in-part of U.S. Ser. No. 10/160,667, filed May 28, 2002, which in turn claims the benefit of U.S. Ser. No. 60/293,488 entitled "METHODS TO IMPROVE CARTILAGE REPAIR SYSTEMS", filed May 25, 2001, U.S. Ser. No. 60/363,527, entitled "NOVEL DEVICES FOR CARTILAGE REPAIR, filed Mar. 12, 2002 and U.S. Ser. Nos. 60/380,695 and 60/380,692, entitled "METHODS AND COMPOSITIONS FOR CARTILAGE REPAIR," and "METHODS FOR JOINT REPAIR," filed May 14, 2002, all of which applications are hereby incorporated by reference in their entireties. U.S. Ser. No. 10/997,407 is also a continuation-in-part of U.S. application Ser. No. 10/681,750 filed Oct. 7, 2003 entitled "MINIMALLY INVASIVE JOINT IMPLANT WITH 3-DIMENSIONAL GEOMETRY MATCHING THE ARTICULAR SURFACES and claims benefit of U.S. provisional patent application 60/467,686 filed May 2, 2003 entitled "JOINT IMPLANTS." Each of the above-referenced applications is incorporated herein by reference in its entirety.

### FIELD OF THE INVENTION

The present invention relates to orthopedic methods, systems and devices and more particularly relates to methods, systems and devices for for treating facet joints, uncovertebral joints, costovertebral joints and other joints.

### BACKGROUND OF THE INVENTION

There are various types of cartilage, e.g., hyaline cartilage and fibrocartilage. Hyaline cartilage is found at the articular surfaces of bones, e.g., in the joints, and is responsible for providing the smooth gliding motion characteristic of moveable joints. Articular cartilage is firmly attached to the underlying bones and measures typically less than 5 mm in thickness in human joints, with considerable variation depending on the joint and the site within the joint.

Adult cartilage has a limited ability of repair; thus, damage to cartilage produced by disease, such as rheumatoid and/or osteoarthritis, or trauma can lead to serious physical deformity and debilitation. Furthermore, as human articular cartilage ages, its tensile properties change. The superficial zone of the knee articular cartilage exhibits an increase in tensile strength up to the third decade of life, after which it decreases markedly with age as detectable damage to type II collagen occurs at the articular surface. The deep zone cartilage also exhibits a progressive decrease in tensile strength with increasing age, although collagen content does not appear to decrease. These observations indicate that there are changes in mechanical and, hence, structural organization of cartilage with aging that, if sufficiently developed, can predispose cartilage to traumatic damage.

Once damage occurs, joint repair can be addressed through a number of approaches. One approach includes the use of matrices, tissue scaffolds or other carriers implanted with cells (e.g., chondrocytes, chondrocyte progenitors, stromal cells, mesenchymal stem cells, etc.). These solutions have been described as a potential treatment for cartilage and meniscal repair or replacement. See, also, International Publications WO 99/51719 to Fofonoff, published Oct. 14, 1999; WO01/91672 to Simon et al., published Dec. 6, 2001; and WO01/17463 to Mannsmann, published Mar. 15, 2001; U.S. Pat. No. 6,283,980 B1 to Vibe-Hansen et al., issued Sep. 4, 2001, U.S. Pat. No. 5,842,477 to Naughton issued Dec. 1, 1998, U.S. Pat. No. 5,769,899 to Schwartz et al. issued Jun. 23, 1998, U. S. Pat. No. 4,609,551 to Caplan et al. issued Sep. 2, 1986, U.S. Pat. No. 5,041,138 to Vacanti et al. issued Aug. 29, 1991, U.S. Pat. No. 5,197,985 to Caplan et al. issued Mar. 30, 1993, U.S. Pat. No. 5,226,914 to Caplan et al. issued Jul. 13, 1993, U.S. Pat. No. 6,328,765 to Hardwick et al. issued Dec. 11, 2001, U.S. Pat. No. 6,281,195 to Rueger et al. issued Aug. 28, 2001, and U.S. Pat. No. 4,846,835 to Grande issued Jul. 11, 1989. However, clinical outcomes with biologic replacement materials such as allograft and autograft systems and tissue scaffolds have been uncertain since most of these materials do not achieve a morphologic arrangement or structure similar to or identical to that of normal, disease-free human tissue it is intended to replace. Moreover, the mechanical durability of these biologic replacement materials remains uncertain.

Usually, severe damage or loss of cartilage is treated by replacement of the joint with a prosthetic material, for example, silicone, e.g. for cosmetic repairs, or metal alloys. See, e.g., U.S. Pat. No. 6,383,228 to Schmotzer, issued May 7, 2002; U.S. Pat. No. 6,203,576 to Afriat et al., issued Mar. 20, 2001; U.S. Pat. No. 6,126,690 to Ateshian, et al., issued Oct. 3, 2000. Implantation of these prosthetic devices is usually associated with loss of underlying tissue and bone without recovery of the full function allowed by the original cartilage and, with some devices, serious long-term complications associated with the loss of significant amount of tissue and bone can include infection, osteolysis and also loosening of the implant.

Further, joint arthroplasties are highly invasive and require surgical resection of the entire articular surface of one or more bones, or a majority thereof. With these procedures, the marrow space is often reamed to fit the stem of the prosthesis. The reaming results in a loss of the patient's bone stock. U.S. Pat. No. 5,593,450 to Scott et al. issued Jan. 14, 1997 discloses an oval domed shaped patella prosthesis. The prosthesis has a femoral component that includes two condyles as articulating surfaces. The two condyles meet to form a second trochlear groove and ride on a tibial component that articulates with respect to the femoral component. A patella component is provided to engage the trochlear groove. U.S. Pat. No. 6,090,144 to Letot et al. issued Jul. 18, 2000 discloses a knee prosthesis that includes a tibial component and a meniscal component that is adapted to be engaged with the tibial component through an asymmetrical engagement.

A variety of materials can be used in replacing a joint with a prosthetic, for example, silicone, e.g. for cosmetic repairs, or suitable metal alloys are appropriate. See, e.g., U.S. Pat. No. 6,443,991 B1 to Running issued Sep. 3, 2002, U.S. Pat. No. 6,387,131 B1 to Miehike et al. issued May 14, 2002; U.S. Pat. No. 6,383,228 to Schmotzer issued May 7, 2002; U.S. Pat. No. 6,344,059 B1 to Krakovits et al. issued Feb. 5, 2002; U.S. Pat. No. 6,203,576 to Afriat et al. issued Mar. 20, 2001; U.S. Pat. No. 6,126,690 to Ateshian et al. issued Oct. 3, 2000; U.S. Pat. No. 6,013,103 to Kaufman et al. issued Jan. 11, 2000. Implantation of these prosthetic devices is usually associated with loss of underlying tissue and bone without recovery of the full function allowed by the original cartilage and, with some devices, serious long-term complications associated with the loss of significant amounts of tissue and bone can cause loosening of the implant. One such complication is osteolysis. Once the prosthesis becomes loosened from the joint, regardless of the cause, the prosthesis will then need to be replaced. Since the patient's bone stock is limited, the number of possible replacement surgeries is also limited for joint arthroplasty.

As can be appreciated, joint arthroplasties are highly invasive and require surgical resection of the entire, or a majority of the, articular surface of one or more bones involved in the repair. Typically with these procedures, the marrow space is fairly extensively reamed in order to fit the stem of the prosthesis within the bone. Reaming results in a loss of the patient's bone stock and over time subsequent osteolysis will frequently lead to loosening of the prosthesis. Further, the area where the implant and the bone mate degrades over time requiring the prosthesis to eventually be replaced. Since the patient's bone stock is limited, the number of possible replacement surgeries is also limited for joint arthroplasty. In short, over the course of 15 to 20 years, and in some cases even shorter time periods, the patient can run out of therapeutic options ultimately resulting in a painful, nonfunctional joint.

U.S. Pat. No. 6,206,927 to Fell, et al., issued Mar. 27, 2001, and U.S. Pat. No. 6,558,421 to Fell, et al., issued May 6, 2003, disclose a surgically implantable knee prosthesis that does not require bone resection. This prosthesis is described as substantially elliptical in shape with one or more straight edges. Accordingly, these devices are not designed to substantially conform to the actual shape (contour) of the remaining cartilage in vivo and/or the underlying bone. Thus, integration of the implant can be extremely difficult due to differences in thickness and curvature between the patient's surrounding cartilage and/or the underlying subchondral bone and the prosthesis. U.S. Pat. No. 6,554,866 to Aicher, et al. issued Apr. 29, 2003 describes a mono-condylar knee joint prosthesis.

Interpositional knee devices that are not attached to both the tibia and femur have been described. For example, Platt et al. (1969) "Mould Arthroplasty of the Knee," Journal of Bone and Joint Surgery 51B(1) :76-87, describes a hemi-arthroplasty with a convex undersurface that was not rigidly attached to the tibia. Devices that are attached to the bone have also been described. Two attachment designs are commonly used. The McKeever design is a cross-bar member, shaped like a "t" from a top perspective view, that extends from the bone mating surface of the device such that the "t" portion penetrates the bone surface while the surrounding surface from which the "t" extends abuts the bone surface. See McKeever, "Tibial Plateau Prosthesis," Chapter 7, p. 86. An alternative attachment design is the Macintosh design, which replaces the "t" shaped fin for a series of multiple flat serrations or teeth. See Potter, "Arthroplasty of the Knee with Tibial Metallic Implants of the McKeever and Macintosh Design," Surg. Clins. Of North Am. 49(4): 903-915 (1969).

U.S. Pat. No. 4,502,161 to Wall issued Mar. 5, 1985, describes a prosthetic meniscus constructed from materials such as silicone rubber or Teflon with reinforcing materials of stainless steel or nylon strands. U.S. Pat. No. 4,085,466 to Goodfellow et al. issued Mar. 25, 1978, describes a meniscal component made from plastic materials. Reconstruction of meniscal lesions has also been attempted with carbon-fiber-polyurethane-poly (L-lactide). Leeslag, et al., Biological and Biomechanical Performance of Biomaterials (Christel et al., eds.) Elsevier Science Publishers B.V., Amsterdam. 1986. pp. 347-352. Reconstruction of meniscal lesions is also possible with bioresorbable materials and tissue scaffolds.

WO 00/19911 A2 discloses a distractor having two handles with respective jaws. Two blades are associated with the jaws, whereby each blade comprises an implant contacting surface and an outwardly facing distracting surface shaped to securely engage the vertebrae being treated.

However, currently available devices do not always provide ideal alignment with the articular surfaces and the resultant joint congruity. Poor alignment and poor joint congruity can, for example, lead to instability of the joint.

Thus, there remains a need for compositions for repair of facet joints, uncovertebral joints, and costovertebral joints, among others. Further, there is a need for an implant or implant system that improves the anatomic result of the joint correction procedure by providing surfaces that more closely resemble the joint anatomy of a patient. Additionally, what is needed is an implant or implant system that provides an improved functional facet, uncovertebral, and costovertebral, joint.

### SUMMARY OF THE INVENTION

In accordance with the present invention, there is provided a method of making a patient-specific surgical instrument as defined in claim 1.

Further advantages are achieved by the embodiments indicated by the dependent claims.

### BRIEF DESCRIPTION OF THE DRAWINGS

FIG. 1A is a block diagram of a method for assessing a joint in need of repair according to the invention wherein the existing joint surface is unaltered, or substantially unaltered, prior to receiving the selected implant. FIG. 1B is a block diagram of a method for assessing a joint in need of repair according to the invention wherein the existing joint surface is unaltered, or substantially unaltered, prior to designing an implant suitable to achieve the repair. FIG. 1C is a block diagram of a method for developing an implant and using the implant in a patient.

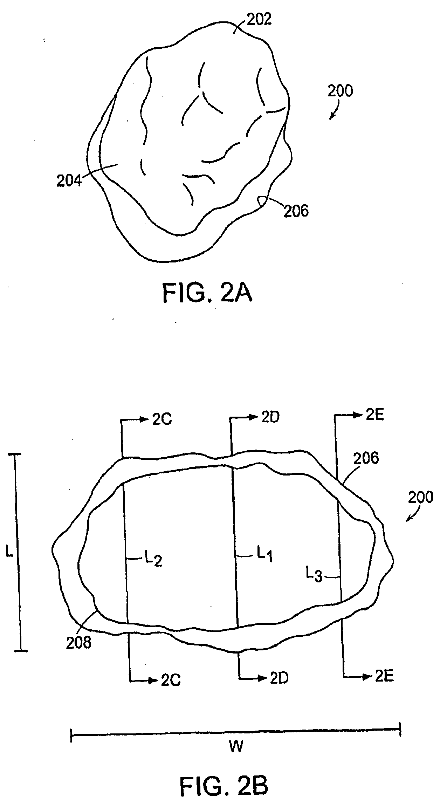

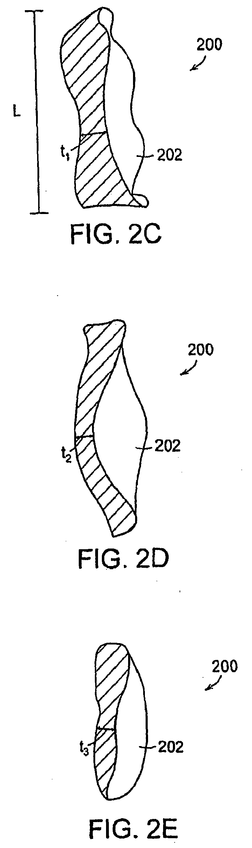

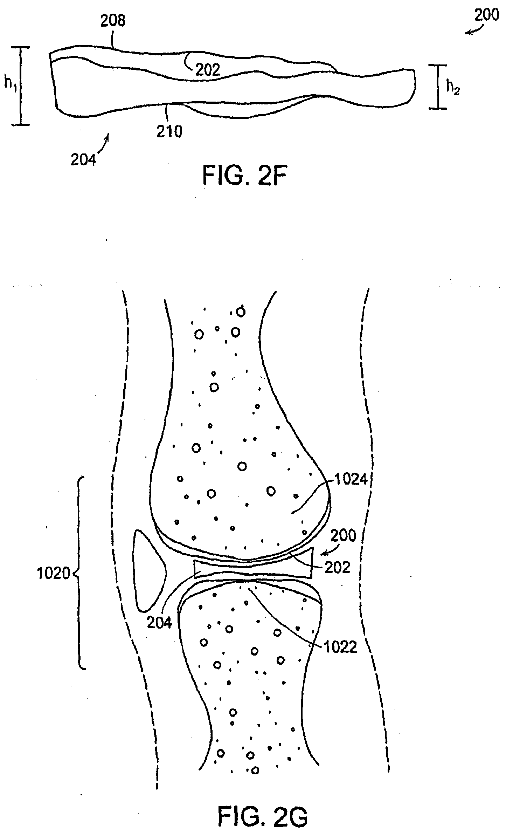

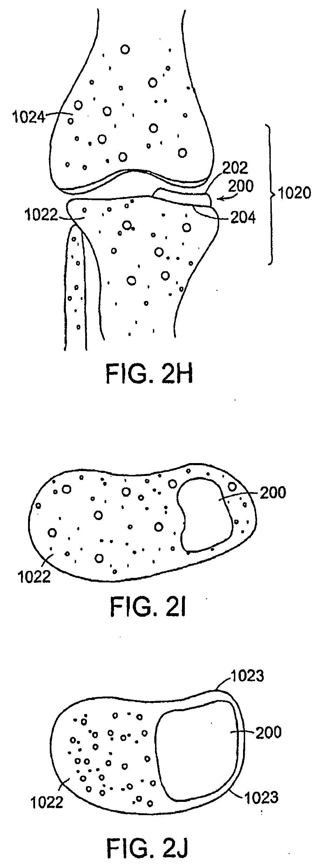

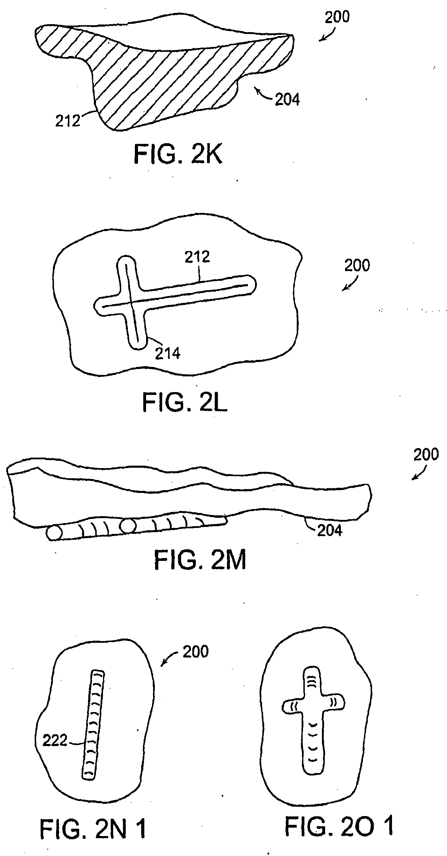

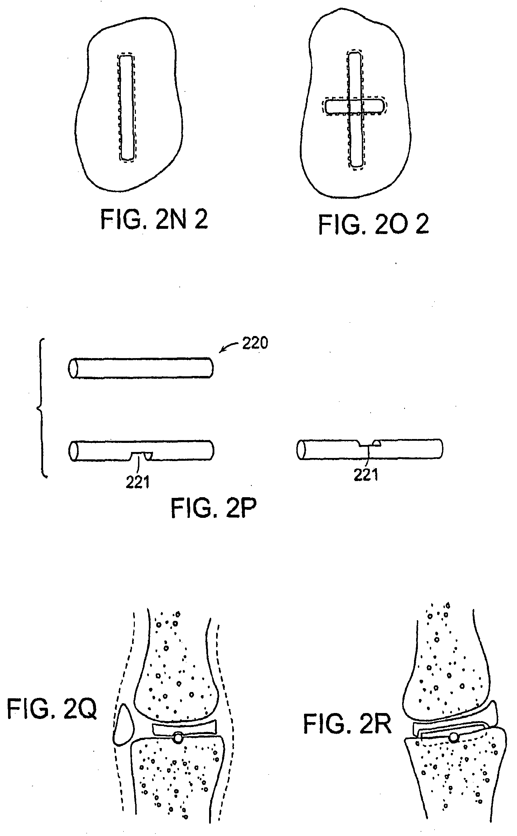

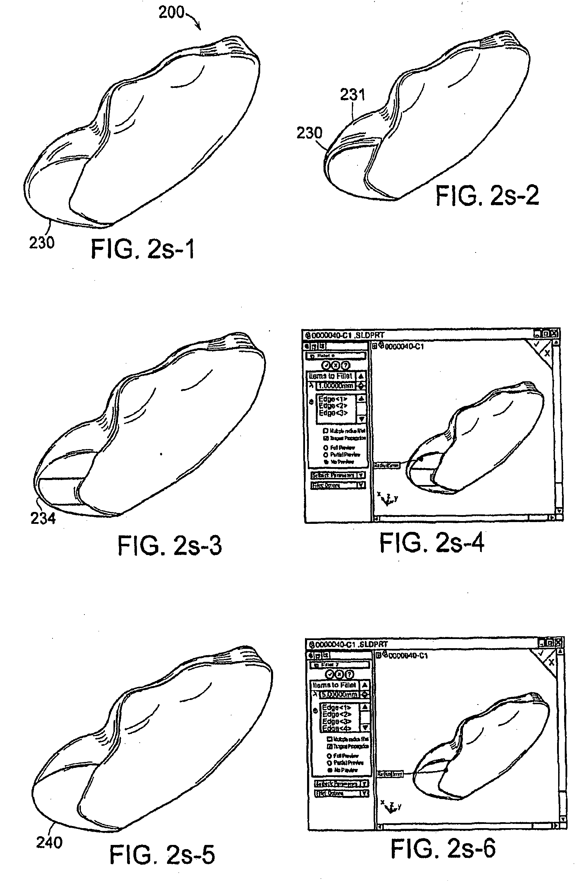

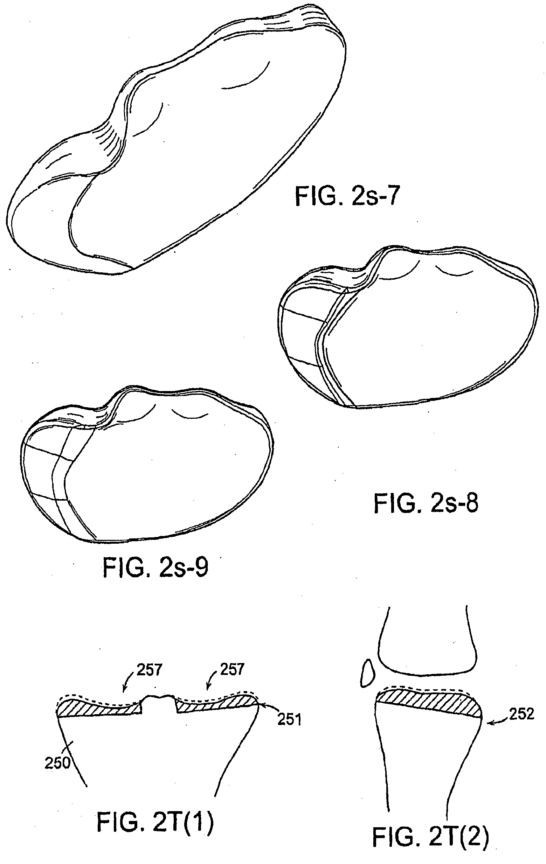

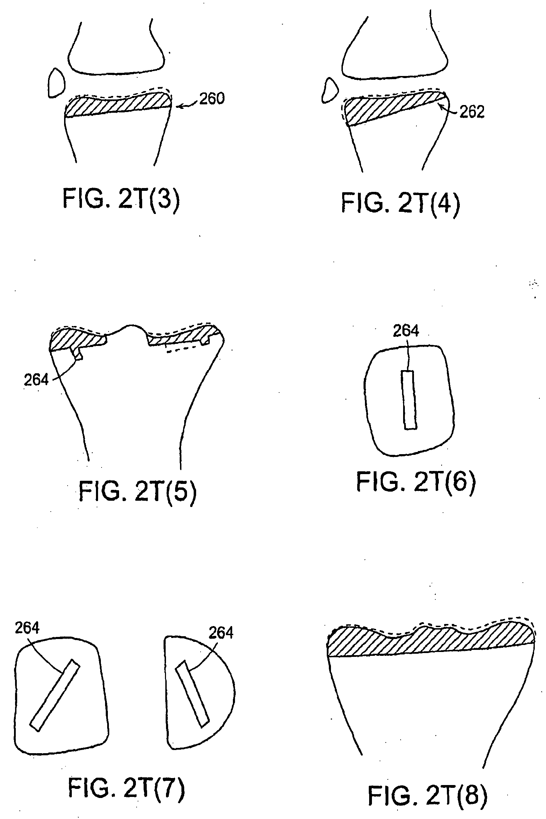

FIG. 2A is a perspective view of a joint implant of the invention suitable for implantation in a joint. FIG. 2B is a top view of the implant of FIG. 2A. FIG. 2C is a cross-sectional view of the implant of FIG. 2B along the lines C-C shown in FIG. 2B. FIG. 2D is a cross-sectional view along the lines D-D shown in FIG. 2B. FIG. 2E is a cross-sectional view along the lines E-E shown in FIG. 2B. FIG. 2F is a side view of the implant of FIG. 2A. FIG. 2G is a cross-sectional view of the implant of FIG. 2A shown implanted taken along a plane parallel to the sagittal plane. FIG. 2H is a cross-sectional view of the implant of FIG. 2A shown implanted taken along a plane parallel to the coronal plane. FIG. 2I is a cross-sectional view of the implant of FIG. 2A shown implanted taken along a plane parallel to the axial plane. FIG. 2J shows a slightly larger implant that extends closer to the bone medially (towards the edge of the tibial plateau) and anteriorly and posteriorly. FIG. 2K is a side view of an alternate embodiment of the joint implant of FIG. 2A showing an anchor in the form of a keel. FIG. 2L is a bottom view of an alternate embodiment of the joint implant of FIG. 2A showing an anchor. FIG. 2M shows an anchor in the form of a cross-member. FIG. 2N-O are alternative embodiments of the implant showing the lower surface have a trough for receiving a cross-bar. FIG. 2P illustrates a variety of cross-bars. FIGS. 2Q-R illustrate the device implanted within a joint. FIGS. **2S( 1-9)** illustrate another implant suitable for the tibial plateau further having a chamfer cut along one edge. FIG. 2T(1- 8) illustrate an alternate embodiment of the tibial implant wherein the surface of the joint is altered to create a flat or angled surface for the implant to mate with.
FIG. 3 is an example of a cross-section of a vertebra demonstrating one normal and one degenerated facet joint.
FIG. 4 is an example of a surgical instrument for removal of bone overgrowth and spurs.
FIG. 5 is an example of a surgical instrument for shaping and smoothing the articular surface.
FIG. 6 is an example of an instrument for shaping a facet or other joint and for inserting an implant. The instrument has a round **601 A** or tapered tip **601 B**.
FIG. 7 is an example of an instrument with a shaver **700.**
FIG. 8 is an example of a distraction device for preparing a joint for implant insertion.
FIG. 9 shows various embodiments for distracting the joint and facilitating implant insertion.
FIG. 10 shows various embodiments describing various types of implant margin, including tapered designs **1001** and round designs **1002.**

### DETAILED DESCRIPTION OF THE INVENTION

The following description is presented to enable any person skilled in the art to make and use the invention. Various modifications to the embodiments described will be readily apparent to those skilled in the art, and the generic principles defined herein can be applied to other embodiments and applications without departing from the scope of the present invention as defined by the appended claims. Thus, the present invention is not intended to be limited to the embodiments shown, but is to be accorded the widest scope consistent with the principles and features disclosed herein.

As will be appreciated by those of skill in the art, methods recited herein may be carried out in any order of the recited events which is logically possible, as well as the recited order of events. Furthermore, where a range of values is provided, it is understood that every intervening value, between the upper and lower limit of that range and any other stated or intervening value in that stated range is encompassed within the invention. Also, it is contemplated that any optional feature of the inventive variations described may be set forth and claimed independently, or in combination with any one or more of the features described herein.

The practice of the present invention can employ, unless otherwise indicated, conventional and digital methods of x-ray imaging and processing, x-ray tomosynthesis, ultrasound including A-scan, B-scan and C-scan, computed tomography (CT scan), magnetic resonance imaging (MRI), optical coherence tomography, single photon emission tomography (SPECT) and positron emission tomography (PET) within the skill of the art. Such techniques are explained fully in the literature and need not be described herein. See, e.g., X-Ray Structure Determination: A Practical Guide, 2nd Edition, editors Stout and Jensen, 1989, John Wiley & Sons, publisher; Body CT: A Practical Approach, editor Slone, 1999, McGraw-Hill publisher; X-ray Diagnosis: A Physician's Approach, editor Lam, 1998 Springer-Verlag, publisher; and Dental Radiology: Understanding the X-Ray Image, editor Laetitia Brocklebank 1997, Oxford University Press publisher. See also, The Essential Physics of Medical Imaging (2 nd Ed.), Jerrold T. Bushberg, et al.

The present invention provides methods and compositions for repairing joints, particularly for repairing articular cartilage and subchondral bone and for facilitating the integration of a wide variety of cartilage and subchondral bone repair materials into a subject. Among other things, the techniques described herein allow for the customization of cartilage or subchondral bone repair material to suit a particular subject, for example in terms of size, cartilage thickness and/or curvature including subchondral bone curvature. When the shape (e.g., size, thickness and/or curvature) of the articular cartilage surface is an exact or near anatomic fit with the non-damaged cartilage or with the subject's original cartilage, the success of repair is enhanced. The repair material can be shaped prior to implantation and such shaping can be based, for example, on electronic images that provide information regarding curvature or thickness of any "normal" cartilage surrounding the defect and/or on curvature of the bone underlying the defect. Thus, the current invention provides, among other things, for minimally invasive methods for partial or complete joint replacement with attached and interpositional designs. The methods will require only minimal or, in some instances, no loss in bone stock. Additionally, unlike with current techniques, the methods described herein will help to restore the integrity of the articular surface by achieving an exact or near anatomic match between the implant and the surrounding or adjacent cartilage and/or subchondral bone.

Advantages of the present invention can include, but are not limited to, (i) optional customization of joint repair, thereby enhancing the efficacy and comfort level for the patient following the repair procedure; (ii) optional eliminating the need for a surgeon to measure the defect to be repaired intraoperatively in some embodiments; (iii) optional eliminating the need for a surgeon to shape the material during the implantation procedure; (iv) providing methods of evaluating curvature of the repair material based on bone or tissue images or based on intraoperative probing techniques; (v) providing methods of repairing joints with only minimal or, in some instances, no loss in bone stock; (vi) improving postoperative joint congruity; (vii) improving the postoperative patient recovery in some embodiments and (viii) improving postoperative function, such as range of motion.

Thus, the methods described herein allow for the design and use of joint repair material that more precisely fits the defect (e.g. site of implantation) or the articular surface(s) and, accordingly, provides improved repair of the joint.

### Assessment of Joints and Alignment

The methods and compositions described herein can be used to treat defects resulting from disease of the cartilage (e.g., osteoarthritis), bone damage, cartilage damage, trauma, and/or degeneration due to overuse or age. The invention allows, among other things, a health practitioner to evaluate and treat such defects. The size, volume and shape of the area of interest can include only the region of cartilage that has the defect, but preferably will also include contiguous parts of the cartilage surrounding the cartilage defect.

As will be appreciated by those of skill in the art, size, curvature and/or thickness measurements can be obtained using any suitable technique. For example, one-dimensional, two-dimensional, and/or three-dimensional measurements can be obtained using suitable mechanical means, laser devices, electromagnetic or optical tracking systems, molds, materials applied to the articular surface that harden and "memorize the surface contour," and/or one or more imaging techniques known in the art. Measurements can be obtained non-invasively and/or intraoperatively (e.g., using a probe or other surgical device). As will be appreciated by those of skill in the art, the thickness of the repair device can vary at any given point depending upon patient's anatomy and/or the depth of the damage to the cartilage and/or bone to be corrected at any particular location on an articular surface.

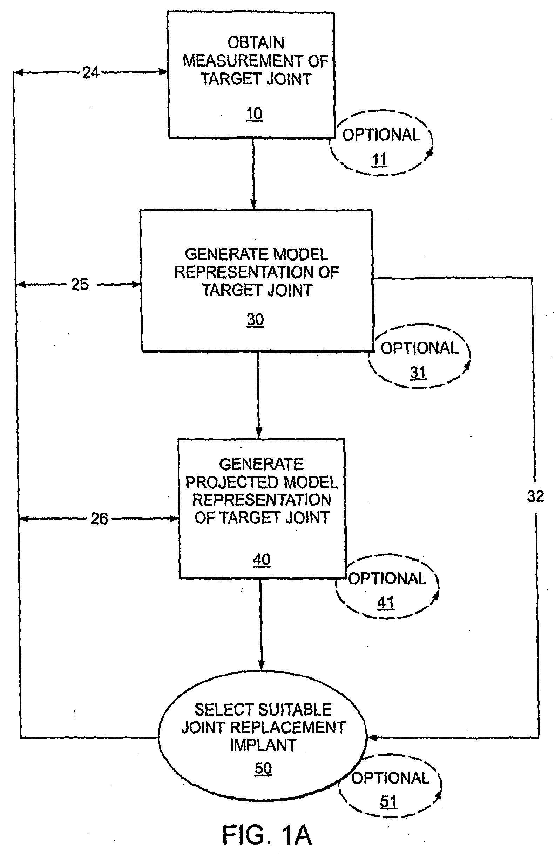

FIG. 1A is a flow chart showing steps taken by a practitioner in assessing a joint. First, a practitioner obtains a measurement of a target joint **10.** The step of obtaining a measurement can be accomplished by taking an image of the joint. This step can be repeated, as necessary, **11** to obtain a plurality of images in order to further refine the joint assessment process. Once the practitioner has obtained the necessary measurements, the information is used to generate a model representation of the target joint being assessed **30.** This model representation can be in the form of a topographical map or image. The model representation of the joint can be in one, two, or three dimensions. It can include a physical model. More than one model can be created **31,** if desired. Either the original model, or a subsequently created model, or both can be used. After the model representation of the joint is generated **30,** the practitioner can optionally generate a projected model representation of the target joint in a corrected condition 40, e.g., from the existing cartilage on the joint surface, by providing a mirror of the opposing joint surface, or a combination thereof. Again, this step can be repeated **41,** as necessary or desired. Using the difference between the topographical condition of the joint and the projected image of the joint, the practitioner can then select a joint implant **50** that is suitable to achieve the corrected joint anatomy. As will be appreciated by those of skill in the art, the selection process **50** can be repeated **51** as often as desired to achieve the desired result. Additionally, it is contemplated that a practitioner can obtain a measurement of a target joint **10** by obtaining, for example, an x-ray, and then select a suitable joint replacement implant **50.**

As will be appreciated by those of skill in the art, the practitioner can proceed directly from the step of generating a model representation of the target joint **30** to the step of selecting a suitable joint replacement implant **50** as shown by the arrow **32.** Additionally, following selection of suitable joint replacement implant **50,** the steps of obtaining measurement of target joint 10, generating model representation of target joint **30** and generating projected model **40,** can be repeated in series or parallel as shown by the flow **24, 25, 26.**

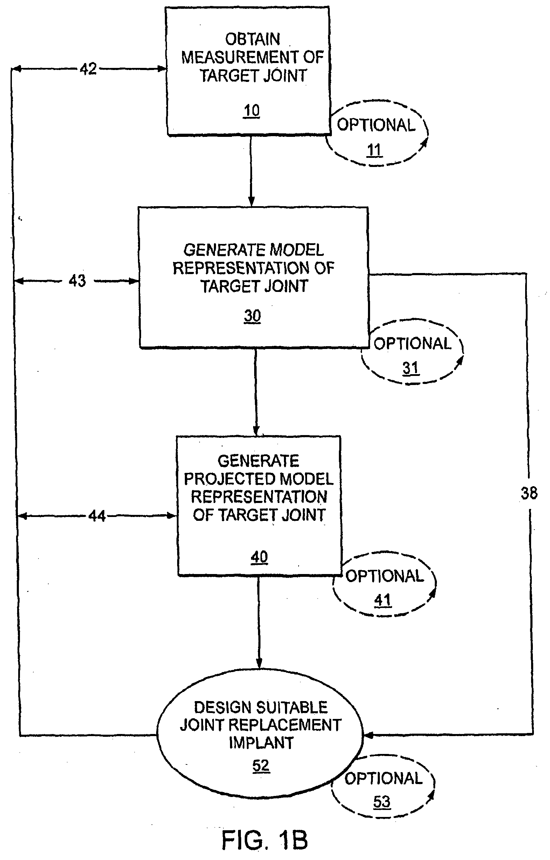

FIG. 1B is an alternate flow chart showing steps taken by a practitioner in assessing a joint. First, a practitioner obtains a measurement of a target joint **10.** The step of obtaining a measurement can be accomplished by taking an image of the joint. This step can be repeated, as necessary, **11** to obtain a plurality of images in order to further refine the joint assessment process. Once the practitioner has obtained the necessary measurements, the information is used to generate a model representation of the target joint being assessed **30.** This model representation can be in the form of a topographical map or image. The model representation of the joint can be in one, two, or three dimensions. The process can be repeated **31** as necessary or desired. It can include a physical model. After the model representation of the joint is assessed **30,** the practitioner can optionally generate a projected model representation of the target joint in a corrected condition **40.** This step can be repeated **41** as necessary or desired. Using the difference between the topographical condition of the joint and the projected image of the joint, the practitioner can then design a joint implant **52** that is suitable to achieve the corrected joint anatomy, repeating the design process **53** as often as necessary to achieve the desired implant design. The practitioner can also assess whether providing additional features, such as rails, keels, lips, pegs, cruciate stems, or anchors, cross-bars, etc. will enhance the implants' performance in the target joint.

As will be appreciated by those of skill in the art, the practitioner can proceed directly from the step of generating a model representation of the target joint **30** to the step of designing a suitable joint replacement implant **52** as shown by the arrow **38.** Similar to the flow shown above, following the design of a suitable joint replacement implant **52,** the steps of obtaining measurement of target joint **10,** generating model representation of target joint **30** and generating projected model **40,** can be repeated in series or parallel as shown by the flow **42, 43,44.**

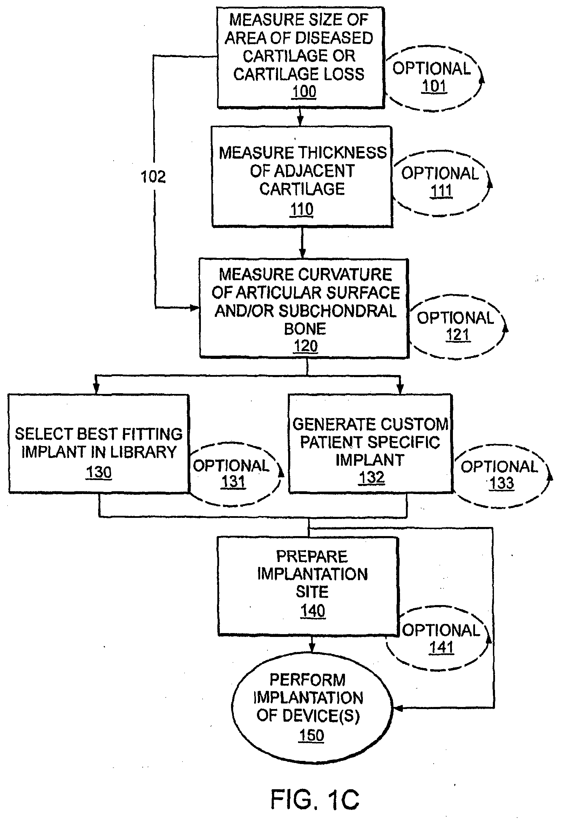

FIG. 1C is a flow chart illustrating the process of selecting an implant for a patient. First, using the techniques described above or those suitable and known in the art at the time the invention is practiced, the size of area of diseased cartilage or cartilage loss is measured **100.** This step can be repeated multiple times **101,** as desired. Once the size of the cartilage defect is measured, the thickness of adjacent cartilage can optionally be measured **110.** This process can also be repeated as desired **111.** Either after measuring the cartilage loss or measuring the thickness of adjacent cartilage, the curvature of the articular surface is then measured **120.** Alternatively, the subchondral bone can be measured. As will be appreciated measurements can be taken of the surface of the joint being repaired, or of the mating surface in order to facilitate development of the best design for the implant surface.

Once the surfaces have been measured, the user either selects the best fitting implant contained in a library of implants **130** or generates a patient-specific implant **132.** These steps can be repeated as desired or necessary to achieve the best fitting implant for a patient, **131, 133.** As will be appreciated by those of skill in the art, the process of selecting or designing an implant can be tested against the information contained in the MRI or x-ray of the patient to ensure that the surfaces of the device achieves a good fit relative to the patient's joint surface. Testing can be accomplished by, for example, superimposing the implant image over the image for the patient's joint. Once it has been determined that a suitable implant has been selected or designed, the implant site can be prepared **140,** for example by removing cartilage or bone from the joint surface, or the implant can be placed into the joint **150.**

The joint implant selected or designed achieves anatomic or near anatomic fit with the existing surface of the joint while presenting a mating surface for the opposing joint surface that replicates the natural joint anatomy. In this instance, both the existing surface of the joint can be assessed as well as the desired resulting surface of the joint. This technique is particularly useful for implants that are not anchored into the bone.

As will be appreciated by those of skill in the art, the physician, or other person practicing the invention, can obtain a measurement of a target joint **10** and then either design **52** or select 50 a suitable joint replacement implant.

### Repair Materials

A wide variety of materials find use in the practice of the present invention, including, but not limited to, plastics, metals, crystal free metals, ceramics, biological materials (e.g., collagen or other extracellular matrix materials), hydroxyapatite, cells (e.g., stem cells, chondrocyte cells or the like), or combinations thereof. Based on the information (e.g., measurements) obtained regarding the defect and the articular surface and/or the subchondral bone, a repair material can be formed or selected. Further, using one or more of these techniques described herein, a cartilage or bone replacement or regenerating material having a curvature that will fit into a particular cartilage defect or onto a particular bone surface, will follow the contour and shape of the articular surface, and will optionally match the thickness of the surrounding cartilage. The repair material can include any combination of materials, and typically includes at least one non-pliable material, for example materials that are not easily bent or changed.

### A. Metal and Polymeric Repair Materials

Currently, joint repair systems often employ metal and/or polymeric materials including, for example, prostheses which are anchored into the underlying bone (e.g., a femur in the case of a knee prosthesis). See, e.g., U.S. Pat. No. 6,203,576 to Afriat, et al. issued Mar. 20, 2001 and U.S. Pat. No. 6,322,588 to Ogle, et al. issued Nov. 27, 2001, and references cited therein. A wide-variety of metals are useful in the practice of the present invention, and can be selected based on any criteria. For example, material selection can be based on resiliency to impart a desired degree of rigidity. Non-limiting examples of suitable metals include silver, gold, platinum, palladium, iridium, copper, tin, lead, antimony, bismuth, zinc, titanium, cobalt, stainless steel, nickel, Iron alloys, cobalt alloys, such as Elgiloy®, a cobalt-chromium-nickel alloy, and MP35N, a nickel-cobalt-chromium-molybdenum alloy, and Nitinol®, a nickel-titanium alloy, aluminum, manganese, iron, tantalum, crystal free metals, such as Liquidmetal® alloys (available from LiquidMetal Technologies, www.liquidmetal.com), other metals that can slowly form polyvalent metal ions, for example to inhibit calcification of implanted substrates in contact with a patient's bodily fluids or tissues, and combinations thereof.

Suitable synthetic polymers include, without limitation, polyamides (e.g., nylon), polyesters, polystyrenes, polyacrylates, vinyl polymer (e.g., polyethylene, polytetrafluoroethylene, polypropylene and polyvinyl chloride), polycarbonates, polyurethanes, poly dimethyl siloxanes, cellulose acetates, polymethyl methacrylates, polyether ether ketones, ethylene vinyl acetates, polysulfones, nitrocelluloses, similar copolymers and mixtures thereof. Bioresorbable synthetic polymers can also be used such as dextran, hydroxyethyl starch, derivatives of gelatin, polyvinylpyrrolidone, polyvinyl alcohol, poly[N-(2-hydroxypropyl) methacrylamide], poly(hydroxy acids), poly(epsilon-caprolactone), polylactic acid, polyglycolic acid, poly(dimethyl glycolic acid), poly(hydroxy butyrate), and similar copolymers can also be used.

Other materials would also be appropriate, for example, the polyketone known as polyetheretherketone (PEEK®). This includes the material PEEK 450G, which is an unfilled PEEK approved for medical implantation available from Victrex of Lancashire, Great Britain. (Victrex is located at www.matweb.com or see Boedeker www.boedeker.com). Other sources of this material include Gharda located in Panoli, India (www.ghardapolymers.com).

It should be noted that the material selected can also be filled. For example, other grades of PEEK are also available and contemplated, such as 30% glass-filled or 30% carbon filled, provided such materials are cleared for use in implantable devices by the FDA, or other regulatory body. Glass filled PEEK reduces the expansion rate and increases the flexural modulus of PEEK relative to that portion which is unfilled. The resulting product is known to be ideal for improved strength, stiffness, or stability. Carbon filled PEEK is known to enhance the compressive strength and stiffness of PEEK and lower its expansion rate. Carbon filled PEEK offers wear resistance and load carrying capability.

As will be appreciated, other suitable similarly biocompatible thermoplastic or thermoplastic polycondensate materials that resist fatigue, have good memory, are flexible, and/or deflectable have very low moisture absorption, and good wear and/or abrasion resistance, can be used without departing from the scope of the invention. The implant can also be comprised of polyetherketoneketone (PEKK).

Other materials that can be used include polyetherketone (PEK), polyetherketoneetherketoneketone (PEKEKK), and polyetheretherketoneketone (PEEKK), and generally a polyaryletheretherketone. Further other polyketones can be used as well as other thermoplastics.

Reference to appropriate polymers that can be used for the implant can be made to the following documents. These documents include: PCT Publication WO 02/02158 A1, dated Jan. 10, 2002 and entitled Bio-Compatible Polymeric Materials; PCT Publication WO 02/00275 A1, dated Jan. 3, 2002 and entitled Bio-Compatible Polymeric Materials; and PCT Publication WO 02/00270 A1, dated Jan. 3, 2002 and entitled Bio-Compatible Polymeric Materials.

The polymers can be prepared by any of a variety of approaches including conventional polymer processing methods. Preferred approaches include, for example, injection molding, which is suitable for the production of polymer components with significant structural features, and rapid prototyping approaches, such as reaction injection molding and stereo-lithography. The substrate can be textured or made porous by either physical abrasion or chemical alteration to facilitate incorporation of the metal coating. Other processes are also appropriate, such as extrusion, injection, compression molding and/or machining techniques. Typically, the polymer is chosen for its physical and mechanical properties and is suitable for carrying and spreading the physical load between the joint surfaces.

More than one metal and/or polymer can be used in combination with each other, For example, one or more metal-containing substrates can be coated with polymers in one or more regions or, alternatively, one or more polymer-containing substrate can be coated in one or more regions with one or more metals.

The system or prosthesis can be porous or porous coated. The porous surface components can be made of various materials including metals, ceramics, and polymers. These surface components can, in turn, be secured by various means to a multitude of structural cores formed of various metals. Suitable porous coatings include, but are not limited to, metal, ceramic, polymeric (e.g., biologically neutral elastomers such as silicone rubber, polyethylene terephthalate and/or combinations thereof or combinations thereof. See, e.g., U.S. Pat. No. 3,605,123 to Hahn, issued Sep. 20, 1971. U.S. Pat. No. 3,808,606 to Tronzo issued May 7, 1974 and U.S. Pat. No. 3,843,975 to Tronzo issued Oct. 29, 1974; U.S. Pat. No. 3,314,420 to Smith issued Apr. 18, 1967, U.S. Pat. No. 3,987,499 to Scharbach issued Oct. 26, 1976; and German Offenlegungsschrift 2,306,552. There can be more than one coating layer and the layers can have the same or different porosities. See, e.g., U.S. Pat. No. 3,938,198 to Kahn, et al., issued Feb. 17,1976.

The coating can be applied by surrounding a core with powdered polymer and heating until cured to form a coating with an internal network of interconnected pores. The tortuosity of the pores (e. g., a measure of length to diameter of the paths through the pores) can be important in evaluating the probable success of such a coating in use on a prosthetic device. See, also, U.S. Pat. No. 4,213,816 to Morris issued Jul. 22, 1980. The porous coating can be applied in the form of a powder and the article as a whole subjected to an elevated temperature that bonds the powder to the substrate. Selection of suitable polymers and/or powder coatings can be determined in view of the teachings and references cited herein, for example based on the melt index of each.

### B. Biological Repair Material

Repair materials can also include one or more biological material either alone or in combination with non-biological materials. For example, any base material can be designed or shaped and suitable cartilage replacement or regenerating material(s) such as fetal cartilage cells can be applied to be the base. The cells can be then be grown in conjunction with the base until the thickness (and/or curvature) of the cartilage surrounding the cartilage defect has been reached. Conditions for growing cells (e.g., chondrocytes) on various substrates in culture, ex vivo and in vivo are described, for example, in U.S. Pat. No. 5,478, 739 to Slivka et al. issued Dec. 26, 1995; U.S. Pat. No. 5,842,477 to Naughton et al. issued Dec. 1, 1998; U.S. Pat. No. 6,283,980 to Vibe-Hansen et al., issued Sep. 4, 2001, and U.S. Pat. No. 6,365,405 to Salzmann et al. issued Apr. 2, 2002. Non-limiting examples of suitable substrates include plastic, tissue scaffold, a bone replacement material (e.g., a hydroxyapatite, a bioresorbable material), or any other material suitable for growing a cartilage replacement or regenerating material on it.

Biological polymers can be naturally occurring or produced in vitro by fermentation and the like. Suitable biological polymers include, without limitation, collagen, elastin, silk, keratin, gelatin, polyamino acids, cat gut sutures, polysaccharides (e.g., cellulose and starch) and mixtures thereof. Biological polymers can be bioresorbable.

Biological materials used in the methods described herein can be autografts (from the same subject); allografts (from another individual of the same species) and/or xenografts (from another species). See, also, International Patent Publications WO 02/22014 to Alexander et al. published Mar. 21, 2002 and WO 97/27885 to Lee published Aug. 7, 1997. In certain embodiments autologous materials are preferred, as they can carry a reduced risk of immunological complications to the host, including re-absorption of the materials, inflammation and/or scarring of the tissues surrounding the implant site.

In one embodiment of the invention, a probe is used to harvest tissue from a donor site and to prepare a recipient site. The donor site can be located in a xenograft, an allograft or an autograft. The probe is used to achieve a good anatomic match between the donor tissue sample and the recipient site. The probe is specifically designed to achieve a seamless or near seamless match between the donor tissue sample and the recipient site. The probe can, for example, be cylindrical. The distal end of the probe is typically sharp in order to facilitate tissue penetration. Additionally, the distal end of the probe is typically hollow in order to accept the tissue. The probe can have an edge at a defined distance from its distal end, e.g. at 1 cm distance from the distal end and the edge can be used to achieve a defined depth of tissue penetration for harvesting. The edge can be external or can be inside the hollow portion of the probe. For example, an orthopedic surgeon can take the probe and advance it with physical pressure into the cartilage, the subchondral bone and the underlying marrow in the case of a joint such as a knee joint. The surgeon can advance the probe until the external or internal edge reaches the cartilage surface. At that point, the edge will prevent further tissue penetration thereby achieving a constant and reproducible tissue penetration. The distal end of the probe can include one or more blades, saw-like structures, or tissue cutting mechanism. For example, the distal end of the probe can include an iris-like mechanism consisting of several small blades. The blade or blades can be moved using a manual, motorized or electrical mechanism thereby cutting through the tissue and separating the tissue sample from the underlying tissue. Typically, this will be repeated in the donor and the recipient. In the case of an iris-shaped blade mechanism, the individual blades can be moved so as to close the iris thereby separating the tissue sample from the donor site.

In another embodiment of the invention, a laser device or a radiofrequency device can be integrated inside the distal end of the probe. The laser device or the radiofrequency device can be used to cut through the tissue and to separate the tissue sample from the underlying tissue.

In one embodiment of the invention, the same probe can be used in the donor and in the recipient. In another embodiment, similarly shaped probes of slightly different physical dimensions can be used. For example, the probe used in the recipient can be slightly smaller than that used in the donor thereby achieving a tight fit between the tissue sample or tissue transplant and the recipient site. The probe used in the recipient can also be slightly shorter than that used in the donor thereby correcting for any tissue lost during the separation or cutting of the tissue sample from the underlying tissue in the donor material.

Any biological repair material can be sterilized to inactivate biological contaminants such as bacteria, viruses, yeasts, molds, mycoplasmas and parasites. Sterilization can be performed using any suitable technique, for example radiation, such as gamma radiation.

Any of the biological materials described herein can be harvested with use of a robotic device. The robotic device can use information from an electronic image for tissue harvesting.

In certain embodiments, the cartilage replacement material has a particular biochemical composition. For instance, the biochemical composition of the cartilage surrounding a defect can be assessed by taking tissue samples and chemical analysis or by imaging techniques. For example, WO 02/22014 to Alexander describes the use of gadolinium for imaging of articular cartilage to monitor glycosaminoglycan content within the cartilage. The cartilage replacement or regenerating material can then be made or cultured in a manner, to achieve a biochemical composition similar to that of the cartilage surrounding the implantation site. The culture conditions used to achieve the desired biochemical compositions can include, for example, varying concentrations. Biochemical composition of the cartilage replacement or regenerating material can, for example, be influenced by controlling concentrations and exposure times of certain nutrients and growth factors.

### Device Design

### A. Cartilage and Bone Models

Using information on thickness and curvature of the cartilage or underlying bone, a physical model of the surfaces of the articular cartilage and of the underlying bone can be created. This physical model can be representative of a limited area within the joint or it can encompass the entire joint. This model can also take into consideration the presence or absence of a meniscus as well as the presence or absence of some or all of the cartilage. For example, in the knee joint, the physical model can encompass only the medial or lateral femoral condyle, both femoral condyles and the notch region, the medial tibial plateau, the lateral tibial plateau, the entire tibial plateau, the medial patella, the lateral patella, the entire patella or the entire joint. The location of a diseased area of cartilage can be determined, for example using a 3D coordinate system or a 3D Euclidian distance as described in WO 02/22014.

In this way, the size of the defect to be repaired can be determined. This process takes into account that, for example, roughly 80% of patients have a healthy lateral component. As will be apparent, some, but not all, defects will include less than the entire cartilage. Thus, in one embodiment of the invention, the thickness of the normal or only mildly diseased cartilage surrounding one or more cartilage defects is measured. This thickness measurement can be obtained at a single point or, preferably, at multiple points, for example 2 point, 4-6 points, 7-10 points, more than 10 points or over the length of the entire remaining cartilage. Furthermore, once the size of the defect is determined, an appropriate therapy (e.g., articular repair system) can be selected such that as much as possible of the healthy, surrounding tissue is preserved.

In other embodiments, the curvature of the articular surface or subchondral bone can be measured to design and/or shape the repair material. Further, both the thickness of the remaining cartilage and the curvature of the articular surface including bone can be measured to design and/or shape the repair material. Alternatively, the curvature of the subchondral bone can be measured and the resultant measurement(s) can be used to either select or shape a cartilage replacement material. For example, the contour of the subchondral bone can be used to re-create a virtual cartilage surface: the margins of an area of diseased cartilage can be identified. The subchondral bone shape in the diseased areas can be measured. A virtual contour can then be created by copying the subchondral bone surface into the cartilage surface, whereby the copy of the subchondral bone surface connects the margins of the area of diseased cartilage. In shaping the device, the contours can be configured to mate with existing cartilage or to account for the removal of some or all of the cartilage.

FIG. 2A shows a slightly perspective top view of a joint implant **200** of the invention suitable for implantation in a joint such as a facet joint, an uncovertebral joint of a costovertebral joint. As shown in FIG. 2A, the implant can be generated using, for example, a dual surface assessment, as described above with respect to FIGS. 1A and B.

The implant **200** has an upper or frontal surface **202,** a lower or posterior surface **204** and, optionally, a peripheral edge **206.** The upper or frontal surface **202** is formed so that it forms a mating surface for receiving the opposing joint surface; in this instance partially concave to receive a femur, although other joints such as a facet joint, an uncovertebral joint or a costovertebral joint are possible. The concave surface can be variably concave such that it presents a surface to the opposing joint surface, e.g. a negative surface of the mating surface of the femur it communicates with. As will be appreciated by those of skill in the art, the negative impression need not be a perfect one.

The upper or frontal surface **202** of the implant **200** can be shaped by any of a variety of means. For example, the upper or frontal surface **202** can be shaped by projecting the surface from the existing cartilage and/or bone surfaces on the articular surface such as a tibial plateau or the surface of a facet joint, or it can be shaped to mirror the femoral condyle in order to optimize the complimentary surface of the implant when it engages the femoral condyle. Alternatively, the superior surface **202** can be configured to mate with an inferior surface of an implant configured for the opposing femoral condyle.

The lower or posterior surface **204** has optionally a convex surface that matches, or nearly matches, the surface of the joint, e.g. a tibial plateau or a facet or uncovertebral or costovertebral joint, such that it creates an anatomic or near anatomic fit with the tibial plateau or other relevant or applicable articular surface. Depending on the shape of the tibial plateau or applicable articular surface, the lower or posterior surface can be partially convex as well. Thus, the lower or posterior surface **204** presents a surface to the tibial plateau or applicable articular surface that fits within the existing surface. It can be formed to match the existing surface or to match the surface after articular resurfacing.

As will be appreciated by those of skill in the art, the convex surface of the lower or posterior surface **204** need not be perfectly convex. Rather, the lower or posterior surface **204** is more likely consist of convex and concave portions that fit within the existing surface of the tibial plateau or the re-surfaced plateau or re-surfaced applicable articular surface. Thus, the surface is essentially variably convex and concave.

FIG. 2B shows a top view of the joint implant of FIG. 2A. As shown in FIG. 2B the exterior shape **208** of the implant can be elongated. The elongated form can take a variety of shapes including elliptical, quasi-elliptical, race-track, etc. However, as will be appreciated the exterior dimension can be irregular thus not forming a true geometric shape, e.g. ellipse. As will be appreciated by those of skill in the art, the actual exterior shape of an implant can vary depending on the nature of the joint defect to be corrected. Thus the ratio of the length L to the width W can vary from, for example, between 0.25 to 2.0, and more specifically from 0.5 to 1.5. As further shown in FIG. 2B, the length across an axis of the implant **200** varies when taken at points along the width of the implant. For example, as shown in FIG. 2B, L₁≠L₂≠L₃.

Turning now to FIGS. 2C-E, cross-sections of the implant shown in FIG. 2B are depicted along the lines of C-C, D-D, and E-E. The implant has a thickness **t1, t2** and **t3** respectively. As illustrated by the cross-sections, the thickness of the implant varies along both its length L and width W. The actual thickness at a particular location of the implant **200** is a function of the thickness of the cartilage and/or bone to be replaced and the joint mating surface to be replicated. Further, the profile of the implant **200** at any location along its length L or width W is a function of the cartilage and/or bone to be replaced.

FIG. 2F is a lateral view of the implant **200** of FIG. 2A. In this instance, the height of the implant **200** at a first end h₁ is different than the height of the implant at a second end h₂. Further the upper edge **208** can have an overall slope in a downward direction. However, as illustrated the actual slope of the upper edge **208** varies along its length and can, in some instances, be a positive slope. Further the lower edge **210** can have an overall slope in a downward direction. As illustrated the actual slope of the lower edge **210** varies along its length and can, in some instances, be a positive slope. As will be appreciated by those of skill in the art, depending on the anatomy of an individual patient, an implant can be created wherein h₁ and h₂ are equivalent, or substantially equivalent without departing from the scope of the invention.

FIG. 2G is a cross-section taken along a sagittal plane in a body showing the implant **200** implanted within a knee joint **1020** such that the lower surface **204** of the implant 200 lies on the tibial plateau **1022** and the femur **1024** rests on the upper surface **202** of the implant **200.** FIG. 2H is a cross-section taken along a coronal plane in a body showing the implant **200** implanted within a knee joint **1020.** As is apparent from this view, the implant **200** is positioned so that it fits within a superior articular surface **224.** As will be appreciated by those of skill in the art, the articular surface could be the medial or lateral facet, as needed.

FIG. 2I is a view along an axial plane of the body showing the implant **200** implanted within a knee joint **1020** showing the view taken from an aerial, or upper, view. FIG. 2J is a view of an alternate embodiment where the implant is a bit larger such that it extends closer to the bone medially, i.e. towards the edge **1023** of the tibial plateau, as well as extending anteriorly and posteriorly.

FIG. 2K is a cross-section of an implant **200** of the invention, e.g. for a facet joint, an uncovertebral or a costovertebral joint, according to an alternate embodiment. In this embodiment, the lower surface **204** further includes a joint anchor **212.** As illustrated in this embodiment, the joint anchor **212** forms a protrusion, keel or vertical member that extends from the lower surface **204** of the implant **200** and projects into, for example, the bone of the joint. As will be appreciated by those of skill in the art, the keel can be perpendicular or lie within a plane of the body.

Additionally, as shown in FIG. 2L the joint anchor **212** can have a cross-member **214** so that from a bottom perspective, the joint anchor **212** has the appearance of a cross or an "x." As will be appreciated by those of skill in the art, the joint anchor **212** could take on a variety of other forms while still accomplishing the same objective of providing increased stability of the implant **200** in the joint. These forms include, but are not limited to, pins, bulbs, balls, teeth, etc. Additionally, one or more joint anchors **212** can be provided as desired. FIGS. 2M and N illustrate cross-sections of alternate embodiments of a dual component implant from a side view and a front view.

In an alternate embodiment shown in FIG. 2M it may be desirable to provide a one or more cross-members **220** on the lower surface **204** in order to provide a bit of translation movement of the implant relative to the surface of the femur or applicable articular surface, or femur implant. In that event, the cross-member can be formed integral to the surface of the implant or can be one or more separate pieces that fit within a groove **222** on the lower surface **204** of the implant 200. The groove can form a single channel as shown in FIG. 2N1, or can have more than one channel as shown in FIG. 2N2. In either event, the cross-bar then fits within the channel as shown in FIGS. **2N1-N2.** The cross-bar members **220** can form a solid or hollow tube or pipe structure as shown in FIG. 2P. Where two, or more, tubes **220** communicate to provide translation, a groove **221** can be provided along the surface of one or both cross-members to interlock the tubes into a cross-bar member further stabilizing the motion of the cross-bar relative to the implant **200.** As will be appreciated by those of skill in the art, the cross-bar member **220** can be formed integrally with the implant without departing from the scope of the invention.

As shown in FIGS. 2Q-R, it is anticipated that the surface of the tibial plateau will be prepared by forming channels thereon to receive the cross-bar members. Thus facilitating the ability of the implant to seat securely within the joint while still providing movement about an axis when the knee joint is in motion.

FIG. 2S(1-9) illustrate an alternate embodiment of implant **200.** As illustrated in FIG. 2S the edges are beveled to relax a sharp corner. FIG. 2S(**1**) illustrates an implant having a single fillet or bevel **230.** As shown in FIG. 2S(2) two fillets **230, 231** are provided and used for the posterior chamfer. In FIG. 2S(3) a third fillet **234** is provided to create two cut surfaces for the posterior chamfer. The chamfer can assist with insertion of the implant: as the implant is advanced into the joint, the chamfer will assist with distracting the joint until the implant is successfully seated *in situ.*

Turning now to FIG. 2S(**4**) a tangent of the implant is deselected, leaving three posterior curves. FIG. 2S(**5**) shows the result of tangent propagation. FIG. 2S(**6**) illustrates the effect on the design when the bottom curve is selected without tangent propagation. The result of tangent propagation and selection is shown in FIG. 2S(7). As can be seen in FIG. 2S(8-9) the resulting corner has a softer edge but sacrifices less than 0.5 mm of joint space. As will be appreciated by those of skill in the art, additional cutting planes can be added without departing from the scope of the invention.

FIG. 2T illustrates an alternate embodiment of an implant **200** wherein the surface of the tibial plateau **250** is altered to accommodate the implant. As illustrated in FIG. 2T(1- 2) the tibial plateau can be altered for only half of the joint surface **251** or for the full surface **252.** As illustrate in FIG. 2T(**3-4**) the posterior-anterior surface can be flat **260** or graded **262.** Grading can be either positive or negative relative to the anterior surface. Grading can also be used with respect to the implants of FIG. 2T where the grading either lies within a plane or a body or is angled relative to a plane of the body. Additionally, attachment mechanisms can be provided to anchor the implant to the altered surface. As shown in FIG. 2T(**5-7**) keels **264** can be provided. The keels **264** can either sit within a plane, e.g. sagittal or coronal plane, or not sit within a plane (as shown in FIG. 2T(**7**)). FIG. 2T(**8**) illustrates an implant which covers the entire tibial plateau. The upper surface of these implants are designed to conform to the projected shape of the joint as determined under the steps described with respect to FIG. 1, while the lower surface is designed to be flat, or substantially flat to correspond to the modified surface of the joint.

The current inventions provide for multiple devices including implants for treating facet joints, uncovertebral joints and costovertebral joints and methods enabling or facilitating this treatment.

An implant can be any device or repair system for treating a facet joint, uncovertebral joint and costovertebral or any other joint.

### Distraction Device

In another embodiment of the invention, a distraction device can be used to facilitate insertion of an implant into a facet joint. A distraction device can be particularly useful for placement of a balloon or an interpositional implant into the facet joint.

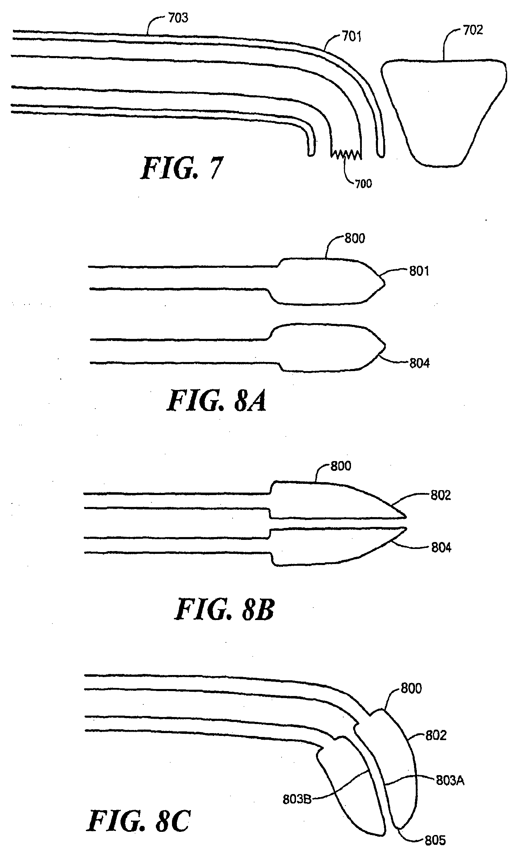

In FIG. 8, for example, the distraction device can include two or more prongs **800.** One or more prongs can be straight **801** (FIG.8A) or curved **802** (FIG. 8B)in one or more dimensions (FIG. 8C). It can be concave **803A** with a mating concave surface **803B.** The curvature can be adapted for a facet joint. It can be tapered in the front **804.** It can also be round at the tip **805.** Preferably, the curvature will be similar to or substantially match that of a facet joint. One or more prongs can be straight or curved, or partially straight and partially curved. Concave and convex shapes are possible can be present at the same time. Irregular shapes can be used.

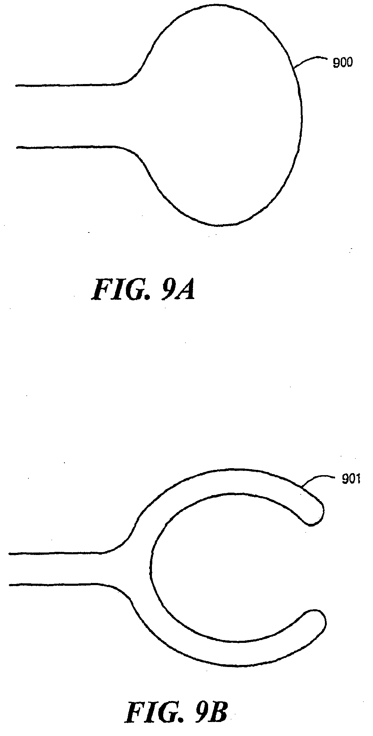

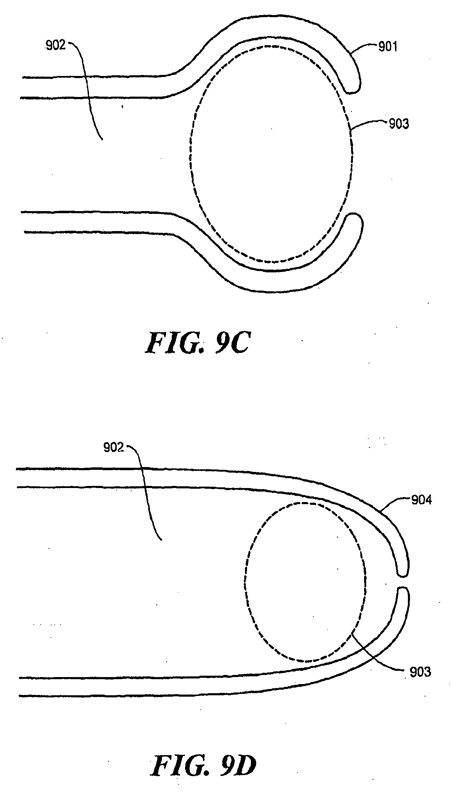

The distraction device can include two plates at the distal tip. In FIG. 9A, the plates can be substantially solid **900.** The plates can also be open on one or more sides **901** (FIG. 9B). The distraction device can have an opening **902** that allows for insertion or placement of an implant **903** after distraction of the joint (FIG. 9C). Various shapes of the distraction device 904 are possible (FIG. 9D). The distance between the plates can be substantially zero at the outset. This facilitates insertion into the joint. Once inserted, the distance between the plates can be increased, for example, using a telescoping or jack or ratchet like mechanism. The distracting mechanism can be located within the joint, preferably between the two plates, or external to the joint, for example near the grip of the device. The two plates can be flat or curved, or partially flat and partially curved. Preferably, the curvature will be similar to or substantially match that of a facet joint. Concave and convex shapes are possible can be present at the same time. Irregular shapes can be used.

The area of the distraction device can be slightly smaller than a facet joint, the same as the facet joint or slightly larger than a facet joint.

The distraction device can be hollow in the center or, alternatively, create a hollow, open space to accept a balloon or an implant.

The distraction device can have an opening in the rear at the side pointing dorsally or externally to allow insertion of the balloon or implant while the distraction device is inside the joint.

The distal portion of the distraction device can be wider, typically between two prongs, than the widest width of the implant in the same dimension, typically supero-inferior, to facilitate removal of the distraction device with the implant remaining in situ.

### Instrument to Remove or Reduce Bone Growth

Degenerated facet joints, uncovertebral joints or costovertebral joints can demonstrate new bone formation, bone remodeling, hypertrophy, bony overgrowth and/or bone spurs. Facet joints, uncovertebral joints or costovertebral joints can enlarge due to new bone formation, bone remodeling, hypertrophy, bony overgrowth and/or bone spurs formation and spur formation. These conditions will be summarized in the term bone growth in the following.

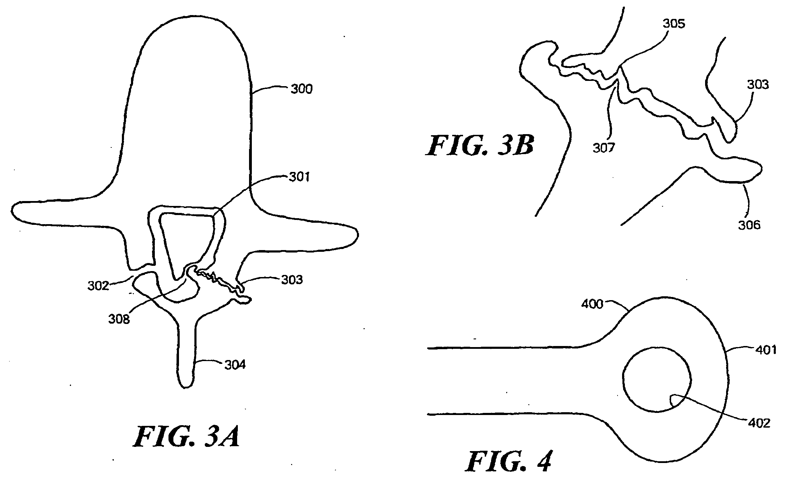

FIGS. **3A** and **B** demonstrate a vertebral body **300,** a thecal sac **301,** which is deformed on the right side by a bone spur **308,** which arises from a facet joint **303.** The facet joint on the right side **303** is degenerated, while the facet joint on the left side **302** is relatively normal in shape still. A spinous process is seen posteriorly **304.** The degenerated facet joint **303** demonstrates multiple peripheral bone spurs **306** which can lead to an enlargement of the joint. There are also irregularities of the articular surface with some deep marks or tracks **305** and ridges or spurs on the articular surface **307.**

Bone growth can cause difficulties during insertion of an implant or balloon. Moreover, bone growth can cause spinal stenosis, including foraminal stenosis, lateral recess stenosis and central stenosis. Thus, while an implant or balloon device designed to alleviate pain originating from the affected joint, i.e. a facet joint, uncovertebral joint or costovertebral joint, the patient may still suffer from back pain and even sciatica after the procedure. The surgeon can optionally consider to reshape the joint and/or remove one or more bone growths.

In one embodiment, an instrument (see **400** in FIG. 4) is used for the reshaping of the joint or the removal of one or more bone growths.

The instrument can, for example, have a ring shape at the tip **401.** The external aspect of the ring can be blunt **401** in order to minimize potential damage to the thecal sac or the nerve roots. The internal portion **402** of the ring can be sharp. All or part of the external portion can be blunt. All or part of the internal portion can be sharp.

The opening of the ring can then be placed over the bone growth and the instrument can be pulled back, thereby removing all or part of the bone growth.

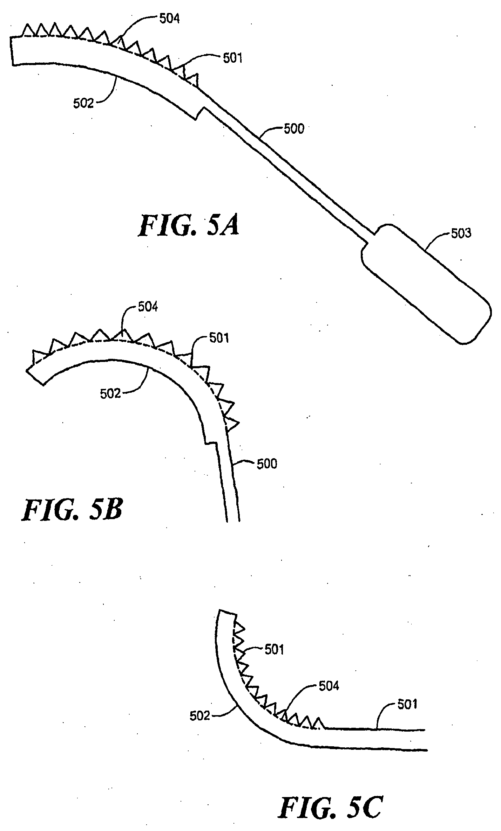

The instrument can include a rough surface creating a rasp-like instrument. In Fig. 5, various embodiments of an instrument **500** with a rough, rasp-like surface **501** are seen. The portion of the implant that inserted into the joint **502** can have different shapes, e.g. convex or concave in one or more planes (FIGS. 5A - 5C). The instrument can have an optional handle 503. The rough surface **501** can be moved over one or two of the articular surfaces of the joint to remove any surface irregularities and to create a new, smooth bearing surface on at least one or, optionally both sides of the joint. The underlying curvature **504** of the rough surface will determine the shape of the articular surface after smoothing it.

Any mechanical device or electrical mechanism capable of removing bone can be utilized in combination with one ore more of the embodiments above and below. For example, in FIG. 7, an instrument with a rotating mill or an oscillating saw or a shaver **700** can be utilized. This instrument can be curved at the tip **701,** thereby protecting the thecal sac 702. The instrument can be in a protective cover **703.** The instrument is typically inserted via a hole in the ligamentum flavum, although it can also be inserted through the joint or both.

The distal portion of the instrument can be tapered, preferably with a rounded tip. The tapered design can facilitate insertion into the joint if the instrument has to be passed through the joint. The rounded or blunt tip can help avoid injury to a nerve root or the thecal sac.

The instrument can be curved in one or more dimensions. One or more convex portions can be included. One or more concave portions can be included. Convex and concave portions can be present in the same device.

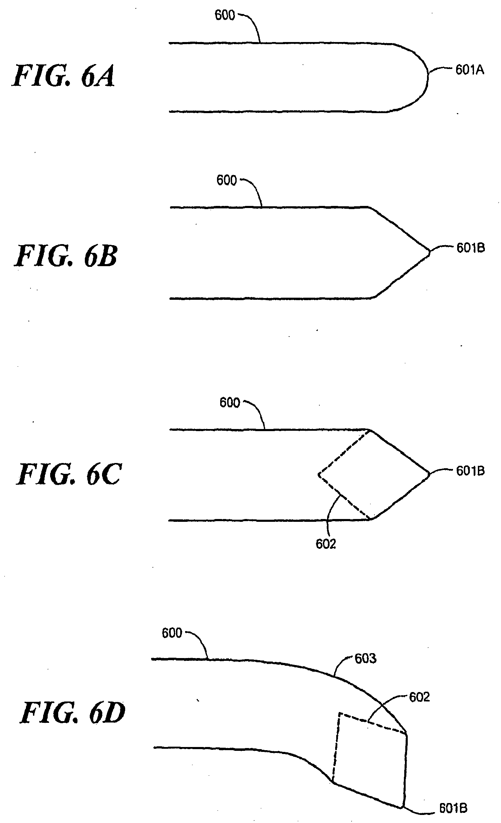

In FIG. 6, an instrument **600** is seen that has a tapered front portion **601.** The tapered front portion can be rounded **601A** (FIG. 6A), or triangular **601B** (FIG. 6B). While the front of the instrument can be tapered, its side portion can optionally have a sharp recess **602** (FIG. 6C and D). The sharp recess can assist in removing some bone overgrowth on or adjacent to the articular surface. The instrument can also be curved **603** near or at its tip **601** (FIG. 6D).

In a preferred embodiment, the instrument shape mirrors the shape of the articular surface.

The instrument can be available in various sizes, thicknesses, lengths and shapes.

In another embodiment, the instrument can have a tip that can be bent backward in an angle equal to or greater than 90 degrees. In this setting, the implant is passed past the bone growth. The tip is then brought in contact with the bone growth and the bone growth is removed.

The instrument can include one or more tubes for suction. Optionally, suction can be performed, also using standard suction devices.

The instrument to remove or reduce bone growth can be used in conjunction with the distraction device. Optionally, both can be integrated.

### Oversized implant or repair device

Degenerated facet joints, uncovertebral joints or costovertebral joints can demonstrate new bone formation, bone remodeling, hypertrophy, bony overgrowth and/or bone spurs. Facet joints, uncovertebral joints or costovertebral joints can enlarge due to new bone formation, bone remodeling, hypertrophy, bony overgrowth and/or bone spurs formation and spur formation. These conditions will be summarized in the term bone growth in the following. Bone growth can lead to an enlargement of the load bearing surface beyond the dimensions of the articular surface, , i.e. portion of the joint that is covered by cartilage prior to the onset of the degenerative and arthritic changes.

Thus, an implant or a repair device including an injectable material sized only to the articular surface, i.e. portion of the joint that is covered by cartilage prior to the onset of the degenerative and arthritic changes, would not cover all of the load bearing surface.

In one embodiment and implant or a repair device including a balloon or an injectable material can be oversized to account for the enlargement of the joint and the greater dimension of the load bearing surface in patients with degenerative or arthritic changes of the facet joints, uncovertebral joints or costovertebral joints. The dimensions of the implant or a repair device including a balloon or an injectable material can be increased in one or more dimensions. In addition, the shape of the implant can be adjusted to account for the bone growth and for irregularities in joint shape as a result of the bone growth.

In another embodiment, the implant size can be selected or adjusted to account for a reduction in size of the joint after removal of a bone growth or to account for a reduction in size of the joint after partial resection of the joint or the articular process.

These adjustments can be made intraoperatively, for example using measuring or sizing devices (see below). Alternatively, these adjustments can be made using imaging software. For example, using CT or MRI data the severity of a spinal stenosis can be estimated. In a second step, resection of a bone growth or partial resection of a joint or articular process can be simulated and it can be determined what the optimal implant size or shape is following these adjustments.

### Implant manufacturing

The implant can be patient specific with each implant custom manufactured, for example using CAD/CAM and rapid prototyping and/or casting techniques. Alternatively, the implant can be selected from a pre-existing library or assortment of implants. The library of implants will typically cover a range of sizes and shapes applicable to most patients and also allowing for oversizing consistent with the embodiment above.

### Pre-Existing Repair Systems

As described herein, repair systems of various sizes, curvatures and thicknesses can be obtained. These repair systems can be catalogued and stored to create a library of systems from which an appropriate system for an individual patient can then be selected. In other words, a defect, or an articular surface, is assessed in a particular subject and a pre-existing repair system having a suitable shape and size is selected from the library for further manipulation (e.g. , shaping) and implantation.

### Image guidance

In various embodiments, imaging techniques can be used for delivering a device. The imaging techniques can include the use of x-rays, CT scans, fluoroscopy, C-arms, CT fluoroscopy, C-arms with CT like cross-sectional reconstruction and MRI. In addition to that, surgical navigation systems, for example using radiofrequency or optical object location and reference means, can be used.

### Sizing tool

In another embodiment of the invention, a sizing tool is used to determine the optimal shape of the implant. The sizing tool can be applied at a first procedure, for example using percutaneous need guidance. Preferably, the sizing tool is used at the time of the procedure for insertion of the therapeutic device into the facet joint, uncovertebral joint or costovertebral joint.

The sizing tool can include various tools for measuring implant dimensions. For example, a ruler or a caliper can be part of the sizing tool. The sizing tool can be used to measure and estimate preferred dimensions of the device, e.g. in superoinferior or mediolateral dimension. It can be used to estimate implant thickness, in one or more locations. The sizing tool can also be used to measure implant curvature.

In one embodiment, the sizing tool can be partially or completely deformable. The sizing tool is inserted into the joint, thereby taking the natural shape of the joint. The sizing tool is then withdrawn from the joint. The resultant shape of the sizing tool is then compared against a library or assortment of premanufactured implants and the best fitting implant with the best match relative to the sizing tool is selected.

In another embodiment, the sizing tool can include a gauge to measure implant dimensions in antero-posterior or supero-inferior or medio-lateral direction or combinations thereof. This gauge can, for example, be a ruler integrated into the sizing tool. The sizing tool is inserted into the joint. The area where the dorsal portion of the articular surface aligns with the first visible tick mark on the ruler indicates typically the preferred implant length.

The sizing tool can also include a gauge in superoinferior or any other dimension.

One or more sizing tools can be used. The sizing tool can include one or more dimensions of one or more of the pre-manufactured implants in the implant library or assortment.

The sizing tool can be available in various shapes. For example, a T or t-shape can provide dimensions in two or more directions. The thickness of the sizing tool can be used to estimate the preferred thickness of the implant.

Sizing tools can be made with various different curvatures and radii, typically resembling the radii of the implant. By inserting sizing tools of different radii, the optimal radius for the implant can be determined.

Alternatively, the implant shape and its radii can be determined using an imaging test.

Alternatively, a trial implant can be used. Trial implants can substantially match the size and shape of the implants in the pre-manufactured library of implants or assortment of implants.

The sizing tool can be malleable and/or deformable.

### Preparing the Joint

In some circumstances it may be desirable to alter the articular surface. For example, the surgeon may elect to flatten the articular surface, to shape it, to increase its curvature or to roughen the articular surface or to remove the cartilage.
The shaping can be advantageous for improving the fit between the implant and the articular surface. Roughening of the articular surface can improve the conformance of the implant to the articular surface and can help reduce the risk of implant dislocation.

Facet joints are frequently rather deformed as a result of progressive degenerative changes with deep marks and tracks distorting the articular surface. When an interpositional implant is used, the marks and tracks can be used for stabilizing the implant on one side. The implant is then typically made to mate with the marks and tracks on one side of the joint thereby achieving a highly conforming surface, and, effectively, a significant constraint to assist with reducing possible implant motion on this side of the joint. The opposing articular surface, however, needs to be minimally constraining in order to enable movement between the implant surface and the opposing articular surface. Thus, the opposing surface can therefore be treated and shaped to remove any marks and tracks and to re-establish a smooth gliding surface. Preferably, the opposing surface will be made to match the smooth surface of the implant on this side.

An instrument for preparing the articular surface can be slightly smaller than a facet, uncovertebral or costovertebral joint, similarly sized or larger in size than the respective joint.

The instrument can be curved or flat. The implant can be curved in more than one dimension.

The instrument can be a rasp or mill-like device. It can be mechanical or electrical in nature.

### Improving Implant Stability

In most embodiments, the device shape and size is substantially matched to one or more articular surface. The implant can fill the space between two opposing articular surfaces partially or completely.

The implant can have extenders outside the articular surface for stabilizing implant position and for minimizing the risk of implant dislocation. Such an extender can be intra- or extra-articular in location. If it is extra-articular, it will typically extend outside the joint capsule.

In one embodiment, the extender can be plate or disc or umbrella shaped, covering at least a portion of the bone outside the articular surface. The extender can be only partially plate or disc or umbrella shaped. The plate or disc or umbrella shaped extender will typically be oriented at an angle to the intra-articular portion of the implant, whereby said angle is substantially different from 180 degrees, more preferred less than 150 degrees, more preferred less than 120 degrees, more preferred less than 100 degrees. In some embodiments, the angle may be equal or less than 90 degrees.

The extender can have a constant or variable radii in one or more dimensions. The extender can be adapted to the patient's anatomy.

If a balloon is used and a self-hardening substance is injected into the balloon, the balloon can have a second, separate portion or a second balloon can be attached, whereby the resultant cavity that will be filled with the self-hardening material can be located outside the articular surface area and can be even external to the joint capsule. Once the self-hardening material is injected, the material has hardened and the balloon has been removed, a lip or ridge or extender can be created in this manner that can help stabilize the resultant repair device against the adjacent bone or soft-tissues.

### Protecting Neural and Other Structures

In another embodiment of the invention, the device or implant can be shaped to protect the neural structures. For example, the ventral portion of the implant can be rounded to avoid any damage to the neural structures in the event the implant moves or subluxes or dislocates anteriorly.

The dorsal and superior and inferior margins can also be rounded in order to avoid damage to neural structures in the event of a subluxation or dislocation into the epidural space. Moreover, a round margin can help minimize chronic wear due to pressure onto the joint capsule.

The margin of the implant can be round along the entire implant perimeter or along a portion of the perimeter.

The implant sidewall can be straight or alternatively, it can be slanted with an angle other than 90 degrees. The implant thickness can vary along the perimeter.

The thickness of the implant can be thinner at the margin than in the center, along the entire implant margin or in portions of the implant margin. The thickness of the implant can be thicker at the margin than in the center, along the entire implant margin or in portions of the implant margin.

### Implant Shape for Easy Insertion

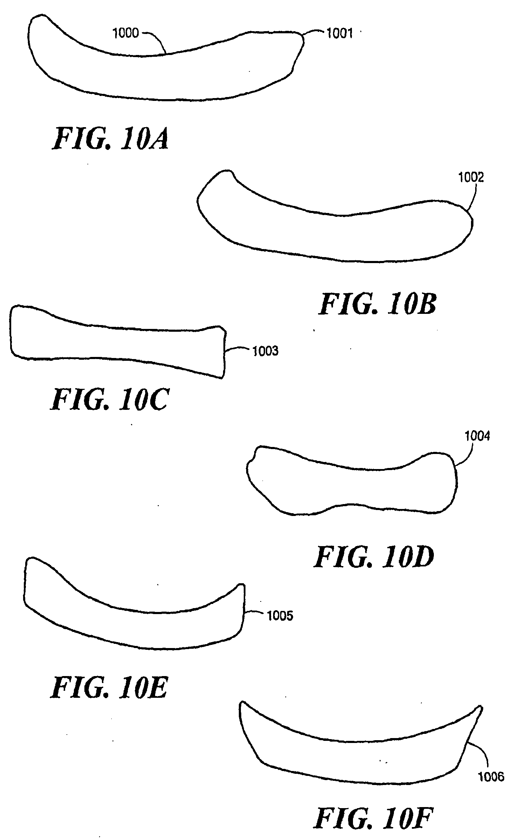

The implant shape can be adapted to facilitate insertion into the joint. For example, in FIG. 10, the portion of the implant 1000 that will face forward, first entering the joint, can be tapered 1001 relative to portions or all of the implant, thereby facilitating insertion. The tapered tip can be pointed **1001** or round **1002** (FIG. 10B). In most embodiments, a round tip is preferably since it can help reduce the risk of damage to adjacent structures.

The implant can have a sharp edge **1003** (FIG. 10C) or a rounded edge **1004** (FIG. 10D). A rounded edge is typically preferred. The implant can have a substantially straight margin **1005** (FIG. 10E) or a substantially tapered margin **1006** (Fig. 10F).

The arthroplasty can have two or more components, one essentially mating with the tibial surface and the other substantially articulating with the femoral component. The two components can have a flat opposing surface. Alternatively, the opposing surface can be curved. The curvature can be a reflection of the tibial shape, the femoral shape including during joint motion, and the meniscal shape and combinations thereof.

Examples of single-component systems include, but are not limited to, a plastic, a polymer, a metal, a metal alloy, crystal free metals, a biologic material or combinations thereof. In certain embodiments, the surface of the repair system facing the underlying bone can be smooth. In other embodiments, the surface of the repair system facing the underlying bone can be porous or porous-coated. In another aspect, the surface of the repair system facing the underlying bone is designed with one or more grooves, for example to facilitate the in-growth of the surrounding tissue. The external surface of the device can have a step-like design, which can be advantageous for altering biomechanical stresses. Optionally, flanges can also be added at one or more positions on the device (e.g., to prevent the repair system from rotating, to control toggle and/or prevent settling into the marrow cavity). The flanges can be part of a conical or a cylindrical design. A portion or all of the repair system facing the underlying bone can also be flat which can help to control depth of the implant and to prevent toggle.

Non-limiting examples of multiple-component systems include combinations of metal, plastic, metal alloys, crystal free metals, and one or more biological materials. One or more components of the articular surface repair system can be composed of a biologic material (e. g. a tissue scaffold with cells such as cartilage cells or stem cells alone or seeded within a substrate such as a bioresorable material or a tissue scaffold, allograft, autograft or combinations thereof) and/or a non-biological material (e.g., polyethylene or a chromium alloy such as chromium cobalt).

Thus, the repair system can include one or more areas of a single material or a combination of materials, for example, the articular surface repair system can have a first and a second component. The first component is typically designed to have size, thickness and curvature similar to that of the cartilage tissue lost while the second component is typically designed to have a curvature similar to the subchondral bone. In addition, the first component can have biomechanical properties similar to articular cartilage, including but not limited to similar elasticity and resistance to axial loading or shear forces. The first and the second component can consist of two different metals or metal alloys. One or more components of the system (e.g., the second portion) can be composed of a biologic material including, but not limited to bone, or a non-biologic material including, but not limited to hydroxyapatite, tantalum, a chromium alloy, chromium cobalt or other metal alloys.

One or more regions of the articular surface repair system (e.g., the outer margin of the first portion and/or the second portion) can be bioresorbable, for example to allow the interface between the articular surface repair system and the patient's normal cartilage, over time, to be filled in with hyaline or fibrocartilage. Similarly, one or more regions (e.g., the outer margin of the first portion of the articular surface repair system and/or the second portion) can be porous. The degree of porosity can change throughout the porous region, linearly or non-linearly, for where the degree of porosity will typically decrease towards the center of the articular surface repair system. The pores can be designed for in-growth of cartilage cells, cartilage matrix, and connective tissue thereby achieving a smooth interface between the articular surface repair system and the surrounding cartilage.

The repair system (e.g., the second component in multiple component systems) can be attached to the patient's bone with use of a cement-like material such as methylmethacrylate, injectable hydroxy-or calcium-apatite materials and the like.

In certain embodiments, one or more portions of the articular surface repair system can be pliable or liquid or deformable at the time of implantation and can harden later. Hardening can occur, for example, within 1 second to 2 hours (or any time period therebetween), preferably with in 1 second to 30 minutes (or any time period therebetween), more preferably between 1 second and 10 minutes (or any time period therebetween).

## Claims

1. A method of making a patient-specific surgical instrument (400, 500, 600, 700) configured to facilitate the placement of an interpositional implant (200) for a facet, an uncovertebral joint or a costovertebral joint of a patient, wherein at least a portion of the surgical instrument (400, 500, 600, 700) has a shape that matches a shape of the facet, uncovertebral or costovertebral joint of the patient, the method comprising deriving the shape of the facet, uncovertebral or costovertebral joint of the patient from electronic image data of the patient; and creating the surgical instrument (400, 500, 600, 700).

2. The method of claim 1, wherein the shape includes an articular shape of the facet, uncovertebral or costovertebral joint of the patient.

3. The method of claim 1 or 2, wherein the patient-specific surgical instrument (400, 500, 600, 700) comprises a distal tip, wherein the distal tip includes two plates (900, 901).

4. The method of claim 3, wherein the two plates (900, 901) are flat or partially flat.

5. The method of claim 3, wherein the two plates (900, 901) are curved or partially curved, having a curvature that substantially matches a curvature of the facet, uncovertebral or costovertebral joint of the patient.

6. The method of claim 3, wherein the patient-specific surgical instrument (400, 500, 600, 700) is configured as a distraction device, wherein a distraction mechanism exists between the two plates (900, 901).

7. The method of claim 1 or 2, wherein the patient-specific surgical instrument (400, 500, 600, 700) comprises two or more prongs (800), wherein one or more prongs (800) are straight or curved.

8. The method of claim 7, wherein the patient-specific surgical instrument (400, 500, 600, 700) comprises two or more prongs (800), wherein one or more prongs (800) are curved, having a curvature adapted for the facet, uncovertebral or costovertebral joint of the patient.

## Patentansprüche

1. Ein Verfahren der Herstellung eines patientenspezifischen chirurgischen Instruments (400, 500, 600, 700), gestaltet zur Vereinfachung der Platzierung eines interpositionalen Implantats (200) für ein Facettengelenk, ein Unkovertebralgelenk oder ein Kostovertebralgelenk eines Patienten, wobei mindestens ein Anteil des chirurgischen Instruments (400, 500, 600, 700) eine Form hat, die zu einer Form des Facetten-, Unkovertebral- oder Kostovertebralgelenks des Patienten passt, das Verfahren umfassend die Ableitung der Form des Facetten-, Unkovertebral- oder Kostovertebralgelenks des Patienten aus elektronischen Bilddaten des Patienten; und die Erstellung der chirurgischen Instrumente (400, 500, 600, 700).

2. Das Verfahren von Anspruch 1, wobei die Form eine artikulare Form des Facetten-, Unkovertebral- oder Kostovertebralgelenks des Patienten einschließt.

3. Das Verfahren von Anspruch 1 oder 2, wobei das patientenspezifische chirurgische Instrument (400, 500, 600, 700) eine distale Spitze umfasst, wobei die distale Spitze zwei Platten (900, 901) einschließt.

4. Das Verfahren von Anspruch 3, wobei die zwei Platten (900, 901) flach oder teilweise flach sind.

5. Das Verfahren von Anspruch 3, wobei die zwei Platten (900, 901) gebogen oder teilweise gebogen sind, mit einer Wölbung, die substantiell zu einer Wölbung des Facetten-, Unkovertebral- oder Kostovertebralgelenks des Patienten passt.

6. Das Verfahren von Anspruch 3, wobei das patientenspezifische chirurgische Instrument (400, 500, 600, 700) als ein Distraktor gestaltet ist, wobei es einen Distraktionsmechanismus zwischen den zwei Platten (900, 901) gibt.

7. Das Verfahren von Anspruch 1 oder 2, wobei das patientenspezifische chirurgische Instrument (400, 500, 600, 700) zwei oder mehr Zinken (800) umfasst, wobei ein oder mehrere Zinken (800) gerade oder gebogen sind.

8. Das Verfahren von Anspruch 7, wobei das patientenspezifische chirurgische Instrument (400, 500, 600, 700) zwei oder mehr Zinken (800) umfasst, wobei ein oder mehrere Zinken (800) gebogen sind, mit einer Wölbung, die für das Facetten-, Unkovertebral- oder Kostovertebralgelenks des Patienten geeignet ist.

## Revendications

1. Procédé de fabrication d'un instrument chirurgical spécifique d'un patient (400, 500, 600, 700) conçu pour faciliter le positionnement d'un implant d'interposition (200) pour une facette vertébrale, une articulation unco-vertébrale ou une articulation costo-vertébrale d'un patient, dans lequel au moins une partie de l'instrument chirurgical (400, 500, 600, 700) a une forme qui correspond à une forme de la facette vertébrale, de l'articulation unco-vertébrale ou de l'articulation costo-vertébrale du patient, le procédé comprenant la déduction de la forme de la facette vertébrale, de l'articulation unco-vertébrale ou de l'articulation costo-vertébrale du patient à partir de données d'image électronique du patient ; et la création de l'instrument chirurgical (400, 500, 600, 700).

2. Procédé selon la revendication 1, dans lequel la forme comprend une forme articulaire de la facette vertébrale, de l'articulation unco-vertébrale ou de l'articulation costo-vertébrale du patient.

3. Procédé selon la revendication 1 ou 2, dans lequel l'instrument chirurgical spécifique du patient (400, 500, 600, 700) comprend une pointe distale, dans lequel la pointe distale comprend deux plaques (900, 901).

4. Procédé selon la revendication 3, dans lequel les deux plaques (900, 901) sont plates ou partiellement plates.

5. Procédé selon la revendication 3, dans lequel les deux plaques (900, 901) sont incurvées ou partiellement incurvées, ayant une courbure qui correspond sensiblement à une courbure de la facette vertébrale, de l'articulation unco-vertébrale ou de l'articulation costo-vertébrale du patient.

6. Procédé selon la revendication 3, dans lequel l'instrument chirurgical spécifique du patient (400, 500, 600, 700) est conçu comme un dispositif de distraction, dans lequel un mécanisme de distraction existe entre les deux plaques (900, 901).

7. Procédé selon la revendication 1 ou 2, dans lequel l'instrument chirurgical spécifique du patient (400, 500, 600, 700) comprend au moins deux dents (800), dans lequel au moins une dent (800) est droite ou incurvée.

8. Procédé selon la revendication 7, dans lequel l'instrument chirurgical spécifique du patient (400, 500, 600, 700) comprend au moins deux dents (800), dans lequel au moins une dent (800) est incurvée, ayant une courbure appropriée pour la facette vertébrale, l'articulation unco-vertébrale ou l'articulation costo-vertébrale du patient.
